Europäisches Patentamt

European Patent Office

Office européen des brevets

(19)

(11)    EP 0 925 504 B1

(12)    **FASCICULE DE BREVET EUROPEEN**

(45) Date de publication et mention
de la délivrance du brevet:
**14.03.2001 Bulletin 2001/11**

(21) Numéro de dépôt: **97919095.6**

(22) Date de dépôt: **12.09.1997**

(51) Int Cl.[7]: **G01N 33/564**, G01N 33/50

(86) Numéro de dépôt international:
**PCT/FR97/01620**

(87) Numéro de publication internationale:
**WO 98/11439 (19.03.1998 Gazette 1998/11)**

(54) **PROCEDE DE DETECTION ET/OU DE QUANTIFICATION D'UN FACTEUR GLIOTOXIQUE**

VERFAHREN ZUR DETEKTION UND/ODER QUANTITATIVEN BESTIMMUNG VON EINEM GLIOTOXISCHEN FAKTOR

METHOD FOR DETECTING AND/OR QUANTIFYING A GLIOTOXIC FACTOR

(84) Etats contractants désignés:
**AT BE CH DE DK ES FI FR GB GR IE IT LI LU MC NL PT SE**

(30) Priorité: **12.09.1996 FR 9611347**

(43) Date de publication de la demande:
**30.06.1999 Bulletin 1999/26**

(73) Titulaire: **BIO MERIEUX
69280 Marcy l'Etoile (FR)**

(72) Inventeurs:
• **MALCUS-VOCANSON, Carine
F-69530 Brignais (FR)**
• **PERRON, Hervé
F-69005 Lyon (FR)**
• **MANDRAND, Bernard
F-69100 Villeurbanne (FR)**

(74) Mandataire: **Guerre, Dominique et al
Cabinet Germain et Maureau,
12, rue Boileau,
BP 6153
69466 Lyon Cedex 06 (FR)**

(56) Documents cités:
**EP-A- 0 667 354          EP-A- 0 731 179**

• **RIEGER ET AL.: "Un facteur gliotoxique et la sclérose en plaques" COMPTES RENDUS DE L'ACADEMIE DES SCIENCES - SERIE III, vol. 319, no. 4, avril 1996, PARIS, pages 343-350, XP000602023**
• **GIULIAN ET AL.: "Secretion of neurotoxins by mononuclear phagocytes infected with HIV-1" SCIENCE, vol. 250, 14 décembre 1990, pages 1593-1596, XP000673668**
• **AMOURI ET AL.: "A new gliotoxic activity and its implications for the immunopathogenesis of multiple sclerosis" NEUROPSYCHIATRIE, vol. 9, no. 2, 1995, MÜNCHEN-DEISENHAFEN, page 94 XP000197520**
• **WARING ET AL.: "Extracellular calcium is not required for gliotoxin or dexamethasone-induced DNA fragmentation: a reappraisal of the use of EGTA." INTERNATIONAL JOURNAL OF IMMUNOPHARMACOLOGY, vol. 17, no. 5, May 1995, OXFORD, pages 403-410, XP000197519**

**Description**

La présente invention concerne la détermination, la détection, et la quantification dans un échantillon biologique d'un facteur gliotoxique, tel qu'associé à la sclérose en plaques.

Par "échantillon biologique", on entend notamment un prélèvement du type fluide biologique, un tissu vivant ou fragment de tissu, une mucosité, un organe ou fragment d'organes, ou tout surnageant de culture obtenu à l'aide d'un prélèvement précité.

Conformément au document WO-A-95/21859, demande déposée au nom de la Demanderesse, on a isolé et/ou caractérisé un facteur cytotoxique vis-à-vis des cellules gliales (astrocytes, oligodendrocytes, microgliocytes), dénommé ci-après facteur gliotoxique. Ce dernier est en particulier associé à la sclérose en plaques, mais pourrait être également associé à d'autres maladies neurodégénératives ou auto-immunes.

A défaut d'un séquençage complet de ce facteur gliotoxique, celui-ci peut être caractérisé, soit à partir d'un procédé (parmi d'autres) permettant de l'isoler ou le purifier, soit à partir de différentes propriétés ou caractères biologiques, biochimiques ou chimiques.

Selon le document WO-A-95/21859, ce facteur gliotoxique est caractérisé par l'ensemble des propriétés ci-après, considérées séparément ou en combinaison :

- il possède une activité toxique vis-à-vis des cellules astrocytaires humaines ou animales, ayant pour effet une désorganisation cytomorphologique de leur réseau de filaments intermédiaires, et/ou une dégradation des protéines de ces filaments intermédiaires et/ou une mort cellulaire notamment par apoptose,
- son activité est associée à au moins une glycoprotéine,
- ce facteur gliotoxique est constitué majoritairement, sinon en totalité, par une fraction légère, centrée sur un poids moléculaire apparent d'environ 17 kD ; cette fraction légère est résistante dans des conditions standard non dénaturantes, à l'action hydrolytique de la pronase, ou de la trypsine, ou de la protéinase K ; et cette même fraction légère présente une forte affinité pour les lectines et notamment la concanavaline-A.

Dès lors, la détection et/ou quantification de ce facteur gliotoxique, dans tout échantillon biologique est un outil intéressant et effectif d'analyse, notamment pour le diagnostic de différentes pathologies, dont la sclérose en plaques, la prédiction, le suivi et la thérapeutique de cette maladie. Toujours selon le document WO-A-95/21859, dont le contenu et la description sont incorporés à la présente demande de brevet par référence, on a décrit et proposé un procédé pour détecter et/ou quantifier dans un échantillon biologique, par exemple un échantillon de liquide céphalo-rachidien (LCR), ce facteur gliotoxique. Un tel procédé comprend au moins les traitements suivants :

- on dispose d'une fraction de départ de cet échantillon, éventuellement enrichie en ledit facteur gliotoxique par tout traitement préalable approprié,
- on incube cette fraction de départ avec un milieu de culture de référence, comprenant par exemple des cellules gliales, notamment immortalisées, par exemple des cellules astrocytaires, et
- on détecte et/ou quantifie les cellules gliales mortes ou vivantes, selon toute technique appropriée, par exemple avec un dosage colorimétrique mettant en oeuvre respectivement la calcéine-AM et l'éthidium homodimère.

Un tel procédé, mis en oeuvre pour la détection et le suivi thérapeutique de la sclérose en plaques suppose un prélèvement dit "invasif", par exemple de LCR, c'est-à-dire requérant un acte préalable médical ou chirurgical.

On a à présent découvert que l'urine s'avère être un fluide biologique particulièrement favorable à la détection de l'activité gliotoxique. Cette découverte était tout à fait surprenante du fait de la composition complexe de l'urine, de son caractère acide, de la présence d'urée qui, a priori, selon les connaissances générales de l'homme du métier, n'étaient pas compatibles avec la détection d'une telle activité cellulaire.

C'est donc de manière totalement inattendue, sur la base de cette découverte, que les inventeurs ont mis au point un procédé de détermination du facteur gliotoxique dans l'urine, qui présente, en outre, l'avantage d'utiliser un prélèvement non invasif, au même titre que tout autre échantillon obtenu de manière "invasive", par exemple un échantillon de LCR, pour la détection et/ou la quantification du dit facteur chez un patient.

Ainsi un premier objet de l'invention est un procédé pour détecter et/ou quantifier, dans un échantillon biologique, un facteur gliotoxique, selon lequel on dispose d'une fraction de départ dudit échantillon, éventuellement enrichie en ledit facteur gliotoxique par un traitement préalable, on incube ladite fraction de départ avec un milieu de culture de référence comprenant des cellules macrogliales, par exemple immortalisées, telles que des cellules astrocytaires, et on détecte et/ou quantifie les cellules macrogliales mortes et/ou vivantes, ledit échantillon biologique étant un échantillon d'urine.

On a ensuite défini un procédé de détection et/ou quantification comprenant une étape de détection et/ou quantification spécifiquement des cellules mortes par l'apoptose induite par le facteur gliotoxique.

Selon le document EP-A-0 731 179, on connaît un procédé de détection de lymphocytes préapoptotiques par la cytométrie de flux, après marquage de l'ADN intracellulaire desdits lymphocytes. Ce document illustre l'utilisation de la cytométrie de flux pour la détection de cellules à l'état préapoptotique non adhérentes, c'est-à-dire en suspension.

Il n'en va pas de même des techniques de détection de l'apoptose sur des cellules adhérentes. Telles que pratiquées aujourd'hui, et en raison de la fragilité des cellules adhérentes apoptotiques, ces techniques reposent sur une observation visuelle de l'apoptose in situ, par microscopie, avec un comptage des cellules apoptotiques. A titre d'exemple, on peut citer l'article de R.W. Keane, A. Srinivasan, L.M. Foster et al., J. Neurosci. Res., 1997, 71, 1992-2003, concernant la détection de cellules adhérentes apoptotiques du système nerveux.

Il s'avérait donc nécessaire de disposer d'un procédé qui pallie les inconvénients précités des techniques couramment utilisées pour la détection de cellules apoptotiques, c'est-à-dire un procédé notamment simple et peu coûteux, et qui de plus permette de prendre en compte les contraintes liées à la fragilité des cellules apoptotiques.

Aussi, selon l'invention, on fournit un procédé pour détecter et/ou quantifier, dans un échantillon biologique, un facteur cytotoxique vis-à-vis de cellules adhérentes cibles et dont la cytotoxicité induit la mort par apoptose desdites cellules, selon lequel on dispose d'une fraction de départ dudit échantillon, éventuellement enrichie en ledit facteur toxique par un traitement préalable, on incube ladite fraction de départ avec un milieu de culture de référence comprenant des cellules adhérentes cibles, et on détecte et/ou quantifie les cellules adhérentes mortes par apoptose, par cytométrie de flux, au moins une caractéristique directe ou indirecte, associée aux cellules adhérentes apoptotiques de tout ou partie du milieu incubé, qui, si elle existe et/ou est quantifiée, qualifie ledit échantillon biologique comme positif, c'est-à-dire contenant ledit facteur toxique.

Le procédé défini ci-dessus est avantageusement appliqué à la détection du facteur gliotoxique et les cellules adhérentes cibles sont les cellules magrogliales, notamment des cellules astrocytaires.

Ainsi selon au moins l'une des caractéristiques précitées, on apporte :

- un procédé pouvant comprendre un prélèvement "non invasif",
- un procédé simple de mise en oeuvre, sensible, spécifique, du facteur gliotoxique.

On sait que l'apoptose des cellules macrogliales est caractérisée notamment par :

* une fragmentation de l'ADN cellulaire des cellules apoptotiques ;
* la conservation de l'intégrité de la membrane cytoplasmique des cellules apoptotiques ;
* des modifications structurales et de taille des cellules apoptotiques.

Grâce au procédé de l'invention, on peut ainsi déterminer :

* la ploïdie des cellules macrogliales, c'est à dire la détermination de la quantité d'ADN restant dans les cellules après extraction des fragments d'ADN, étant entendu que dans des cellules non apoptotiques la quantité d'ADN est conservée puisqu'il n'y a pas de fragmentation ; à partir de la ploïdie, on peut également étudier le cycle cellulaire ;
* l'état des cellules macrogliales ; ainsi, la morphologie cellulaire est déterminée par la taille des cellules, par exemple selon la technique dite FALS ("Forward Light Angle Scatter"), la mort par apoptose des astrocytes entraînant une diminution de leur taille ; par la structure desdites cellules, par exemple selon la technique dite SS ("Side Scatter") ; et éventuellement par la présence et/ou quantité d'une protéine permettant de discriminer les sous-populations cellulaires, telle que la protéine acide fibrillaire gliale (GFAP), par exemple avec un anticorps marqué anti-GFAP identifiable directement ou indirectement.

Aux fins de la détection et/ou quantification des cellules macrogliales mortes par apoptose spécifiquement, par cytométrie de flux, ledit procédé de l'invention comprend au moins un des protocoles (1) à (3) suivants ; avantageusement les protocoles (1) et (2) ou (1) et (3) sont employés en combinaison.

Protocole 1)

- décollement des cellules macrogliales adhérentes,
- traitement des cellules ainsi décollées par un agent de fixation cellulaire et de perméabilisation de la membrane cytoplasmique desdites cellules,
- extraction de fragments d'ADN intracellulaire résultant de l'apoptose,
- marquage de l'ADN cellulaire restant, par un marqueur approprié, et

- détection par cytométrie de flux de la ploïdie des cellules macrogliales.

Avantageusement, l'agent de fixation cellulaire et de perméabilisation de la membrane cytoplasmique des cellules est l'éthanol et le marqueur de l'ADN est l'iodure de propidium (IP).

Protocole (2)

- induction d'une nécrose sur un échantillon de cellules macrogliales vivantes, différent de l'échantillon à analyser, notamment par incubation,
- marquage des débris d'ADN résultant de la nécrose, lesdits débris étant associés à des fragments de la membrane cytoplasmique des cellules macrogliales nécrotiques, le marquage étant effectué après décollement desdites cellules,
- localisation en cytométrie de flux, des cellules mortes par nécrose, et réglage approprié du cytomètre permettant lors de l'étape de détection des cellules mortes par apoptose, d'exclure les cellules mortes par nécrose,
- détection des cellules mortes par apoptose.

Protocole (3)

- partage des cellules de l'échantillon biologique en deux parties égales, et décollement des cellules,
- détection, par cytométrie de flux, dans une des deux parties, des cellules macrogliales mortes par apoptose ou par nécrose, d'une part, et/ou des cellules macrogliales vivantes, d'autre part, étape selon laquelle on traite ladite fraction avec un agent de fixation cellulaire et de perméabilisation de la membrane cytoplasmique ; on extrait les fragments d'ADN ; on marque à l'aide d'un marqueur de l'ADN intracellulaire, et on détecte l'ADN intracellulaire restant,
- détection, par cytométrie de flux après fixation sans extraction, dans l'autre des deux parties, des cellules macrogliales vivantes et des cellules macrogliales apoptotiques, d'une part, et/ou des cellules macrogliales nécrotiques, d'autre part,
- déduction des détections effectuées sur chacune des deux parties, de la quantité de cellules macrogliales apoptotiques.

Selon le protocole (2) ou (3) défini ci-dessus, il est possible de distinguer les cellules apoptotiques de cellules nécrotiques. On sait que les phénomènes de nécrose se matérialisent par un éclatement de la paroi cellulaire et le relargage des constituants de la cellule. Les cellules nécrotiques se distinguent donc des autres cellules, vivantes ou apoptotiques, en ce que l'intégrité de leur membrane cytoplasmique n'est pas conservée. Ceci peut être mis en évidence soit à partir d'une expérience isolée après induction de la nécrose sur des cellules macrogliales vivantes afin de localiser les cellules nécrotiques obtenues et de réaliser le réglage approprié du cytomètre de façon à pouvoir, dans la détermination finale des cellules apoptotiques, exclure ces cellules nécrotiques (protocole 2) ; soit par une étude parallèle sur deux sous-populations identiques issues de mêmes cellules macrogliales après induction potentielle de l'apoptose sur ces cellules, lesdites cellules étant traitées de deux façons distinctes : à savoir, d'une part, après fixation à l'éthanol et extraction des fragments d'ADN, le marquage à l'IP permet de distinguer les cellules vivantes des cellules mortes (nécrose et apoptose) ; d'autre part, après fixation sans extraction des fragments d'ADN, le marquage à l'IP permet ainsi de distinguer les cellules nécrotiques des cellules vivantes ou apoptotiques (puisque, en l'absence d'extraction, les fragments d'ADN sont conservés à l'intérieur des cellules apoptotiques) ; et ensuite, par déduction, on peut déterminer la proportion de chaque sous-catégorie cellulaire, donc la proportion de cellules apoptotiques (protocole 3).

Cette distinction entre cellules nécrotiques et apoptotiques est nécessaire notamment dans le cas d'interférences médicamenteuses chez des patients, lesdites substances pouvant induire des phénomènes de recouvrement de la population des cellules nécrotiques et de la population des cellules apoptotiques, matérialisés en particulier par la génération de résultats faussement positifs au test de détection d'apoptose. Cette interférence a en effet été observée à partir d'échantillons biologiques, par exemple des urines, issus de patients ayant subi un traitement par des substances médicamenteuses interférentes, par exemple le méthotrexate ou le dextropropoxyphène.

L'application du procédé de l'invention pour détecter et/ou quantifier spécifiquement des cellules macrogliales apoptotiques, à un échantillon d'urine, permet d'obtenir un bio-essai particulièrement simple, sensible et spécifique du facteur gliotoxique, par exemple en ce qui concerne l'aide au diagnostic de la sclérose en plaques, qui aujourd'hui requiert beaucoup d'essais ou examens différents sur une période de temps qui peut être relativement longue.

Selon une mise en oeuvre particulière du procédé de l'invention, celui-ci peut comprendre, avant l'étape de détection et/ou quantification des cellules macrogliales apoptotiques, une étape de détection de la viabilité cellulaire. A cet effet, on inocule la fraction de départ dans le milieu de culture de référence, puis on observe le taux de prolifération des cellules macrogliales par rapport à une valeur de référence, en deçà de laquelle ladite fraction dite positive est retenue pour être soumise ensuite à l'étape de détection de l'apoptose des cellules macrogliales spécifiquement, pour qualifier ou non l'échantillon biologique comme vrai positif.

Au cours de cette étape de détection de la viabilité cellulaire, le taux de prolifération des cellules macrogliales peut être obtenu par coloration mitochondriale des cellules macrogliales du milieu de culture, par exemple par du bromure de méthyltétrazolium, puis par mesure de la densité optique du milieu de culture coloré. A titre d'exemple on peut citer le dosage colorimétrique au méthyl-tétrazolium des cellules vivantes (MTT), tel que décrit dans le document WO-A-95/21859.

Pendant l'étape de détection de l'apoptose des cellules macrogliales spécifiquement, on peut avantageusement pratiquer, en outre, une détermination de la présence et/ou quantité d'une protéine permettant de discriminer les sous-populations cellulaires, telle que la protéine acide fibrillaire gliale (GFAP), par exemple avec un anticorps anti-GFAP identifiable directement ou indirectement.

Une détermination des changements intervenant au niveau des phospholipides de la membrane cytoplasmique des cellules apoptotiques, sans affecter son intégrité, et mis en évidence par l'annexine V peut aussi être prévue pour compléter le procédé de l'invention.

La fraction de départ de l'échantillon biologique dans lequel on détecte et/ou quantifie le facteur gliotoxique peut être obtenue par enrichissement ou purification de l'échantillon biologique en facteur gliotoxique. Cet enrichissement ou purification est notamment réalisé par au moins l'un des traitements suivants, à savoir précipitation de la fraction protéique, par exemple avec du sulfate d'ammonium, chromatographie par exclusion et/ou ionique, électrophorèse à une ou deux dimensions, mise en contact avec la protéine A ou une lectine, par exemple la concanavaline-A.

Le milieu de culture de référence dans lequel ladite fraction de départ est incubée comprend de préférence une lignée cellulaire immortalisée de cellules macrogliales, par exemple une lignée cellulaire immortalisée d'astrocytes.

Les cellules macrogliales considérées selon la présente invention comprennent notamment, les astrocytes et les oligodendrocytes.

La présente invention est maintenant décrite par référence aux exemples 1 à 7 suivants et au dessin dans lequel :

- la figure 1 (A à G) représente les histogrammes obtenus par cytométrie de flux pour un échantillon contenant seulement quelques cellules nécrotiques mais ne contenant pas de cellules apoptotiques,
- la figure 2 (A à G) représente les histogrammes obtenus par cytométrie de flux pour un échantillon contenant des cellules apoptotiques.

Les lettres A à L apparaissant sur les histogrammes correspondent à la localisation des cellules visualisées en cytométrie de flux.

Exemple 1: Etape de détection de l'apoptose, par cytométrie de flux.

La lignée d'astrocytes immortalisés est obtenue conformément à l'article de E. Galiana, I. Borde, P. Marin, M. Rassoulzadegan, F. Chuzin, F. Gros, P. Rouget et C. Evrard, Establishment of permanent astroglial cell lines, able to differentiate in vitro, from transgenic mice carrying the polyoma virus large-T gene : an alternative approach to brain cell immortalization, Journal of Neuroscience Research, 1990 ; 26 ; 269-277, article dont le contenu est incorporé par référence à la présente description.

Le milieu dénommé milieu M est constitué comme suit : DMEM (400 µl/200 ml) / F12 (1/1) + 10% FCS non décomplémenté + Pénicilline/Streptomycine (antibiotique) + Fungizone (antifongique) (20 µl/40 ml).

Le cytomètre utilisé est un cytomètre Epics XL Coulter (de la société Coultronics France). Le réglage du programme utilisé est le suivant :

débit : 60 µl/min
gaine liquide : IsoFlow (nom commercial)
fluorescence :

| | tension (V) | gain |
|---|---|---|
| FL1 (FITC) | 605 | 1 |

(suite)

|  | tension (V) | gain |
|---|---|---|
| FL3 (IP) | 715 | 2 |
| AUX (FL3) | 190 | 1 |
| FS | 423 | 1 |
| SS | 800 | 1 |

FL : fluorescence ; FITC : isothiocyanate de fluorescéine ; IP : iodure de propidium ; AUX : canal auxiliaire permettant un double réglage sur l'iodure de propidium ; FS : diffraction ; SS : réfraction à 90°.

Protocole expérimental :

On prépare à l'avance des boîtes 24 ou 48 puits revêtues de poly L-Lysine à 12,5 µg/ml :

En atmosphère stérile (flux laminaire), on effectue les manipulations suivantes :

diluer les tubes de 4 ml de poly-L-Lysine (congelé -20°C) au 1/10ème dans $H_2O$ distillée,
ajouter 250 µl/puits de cette solution,
laisser incuber 2 heures au moins,
aspirer,
rincer avec 500 µl/puits $H_2O$ distillée (24 puits) ou 250 µl/puits (48 puits),
aspirer,
laisser sécher les boîtes ouvertes sous le flux (de quelques min à 30 min),
les refermer et les stocker sous le flux.

Premier jour :

Les cellules contenues dans une boîte 250 cm$^2$ sont trypsinées (solution préparée à partir d'une solution de trypsine 1/250e (S1 : 45 ml) et d'une solution 1% EDTA (S2 : 5 ml) et conservée à +4°C) :

La boîte est sortie de l'étuve. Le milieu qui recouvre les cellules est aspiré (pipette de 10 ml) et éliminé. Les cellules adhérentes sont rincées avec 10 à 15 ml de DMEM/F12 (1/1) qui sont aspirés. On additionne 1 à 1,2 ml de trypsine/EDTA, les cellules sont remises dans l'étuve pendant 10 min. Elles sont décollées de leur support mécaniquement à l'aide d'une pipette de 10 ml de milieu M. Après homogénéisation elles sont lavées dans 20 ml de DMEM/F12 supplémentaires puis centrifugées 10 min à 1500 tours/min (18°C).

Le surnageant est éliminé avec précaution. Les cellules sont reprises par 2 ml de DMEM/F12 et homogénéisées longuement. Elles sont à nouveau lavées dans 25 ml de DMEM/F12 puis centrifugées (10 min, 1500 tours/min, 18°C). Le surnageant est éliminé. Les cellules sont reprises par 2 ml du milieu et homogénéisées longuement afin d'obtenir une suspension la plus uniforme possible. 1 à 5 ml du milieu sont additionnés en fonction de l'importance du culot.

Les cellules sont numérées directement dans une cellule de Tomas. La concentration cellulaire est ajustée à 10 000 cellules/ml par dilution dans le milieu M afin de déposer 20 000 cellules/puits, soit 2 ml/puits pour une plaque à 6 puits. A titre d'exemple pour 8 plaques 6 puits, on a besoin de 8 x 6 x 2 ml de suspension soit 96 ml ; on prépare 120 ml (3 x 40 ml) de suspension ; il reste ainsi environ 25 ml de suspension qui serviront à remettre les cellules restantes en culture, en les ajoutant dans ces 25 ml restant.

Les plaques ainsi préparées sont recouvertes de film protecteur pour éviter l'évaporation.

Deuxième jour :

Le facteur gliotoxique est ajouté dans chaque puits à raison de 60 à 80 µl/puits suivant la quantité présumée de facteur toxique dans l'échantillon (10 µl/puits pour les urines, 10 à 50 µl/puits pour les fractions purifiées et les autres échantillons).

La boîte est sortie de l'étuve. Le film protecteur est enlevé. Le facteur est ajouté en triplicat dans 2 ou 3 puits successifs. Un nouveau film protecteur est mis en place. Les plaques sont remises à l'étuve (37°C $CO_2$ 5% $H_2O$ 95%) pendant 72 heures.

Cinquième jour :

L'état des cellules vivantes présentes dans les puits est évalué par cytométrie de flux après marquage à l'iodure de propidium pour mettre en évidence les cellules apoptotiques.

Les boîtes sont sorties de l'étuve. Le film protecteur est ôté. Le milieu est aspiré (pipettes pasteur droites non cotonnées) en prenant soin de ne pas aspirer les cellules adhérentes au fond des puits. Les cellules sont rincées avec 2 ml de PBS.

Les cellules sont ensuite décollées de manière douce (non enzymatique) par incubation avec une solution de PBS EDTA (1 à 2 ml/puits) (37°C $CO_2$ 5% $H_2O$ 95% pendant 10 min) puis dissociées mécaniquement. Un tel traitement permet de préserver les cellules adhérentes et notamment les cellules apoptotiques. Les puits sont rincés au PBS afin de récupérer les dernières cellules au fond de ceux-ci. 2 lavages en PBS sont réalisés.

Les cellules sont fixées à l'éthanol 70% ce qui génère la formation de pores dans la membrane cellulaire et induit sa perméabilisation. A cet effet, 2 ml/puits d'éthanol 70% (congelé) sont rajoutés. Les cellules sont fixées 1 heure à -20°C. Après une centrifugation pour éliminer l'alcool, un lavage en PBS est réalisé.

Les cellules ainsi perméabilisées sont traitées afin d'extraire les fragments d'ADN générés par l'apoptose. Le tampon d'extraction est préparé extemporanément :

9 ml $Na_2HPO_4$ 0,05M
1 ml acide citrique 25 mM
0,1 % Triton X-100.

Le culot cellulaire obtenu à l'étape suivante est repris par 2 ml de ce tampon, et incubé 1 heure à +4°C.

Après une centrifugation pour éliminer le tampon, un lavage en PBS est réalisé.

Eventuellement, afin de visualiser l'aspect des cellules et leurs caractéristiques astrocytaires, un marquage anti-GFAP peut être réalisé. Dans ce cas, le culot cellulaire est repris par 50 µl de BSA à 5% + 50 µl de saponine à 1% en PBS + 20 µl d'anticorps anti-GFAP (préparé chez le lapin) dilué au 1/100e puis incubé 30 min à température ambiante à l'obscurité. Après 2 lavages en PBS le contremarquage est réalisé (addition de 50 µl de BSA à 5% + 50 µl de saponine à 1% en PBS + 20 µl d'anti-IgG de lapin marqué à la fluorescéine (FITC) dilué au 1/10e), incubation 15 min à température ambiante à l'obscurité. 2 lavages en PBS sont réalisés.

Après l'éventuel marquage GFAP les suspensions peuvent être conservées 1 à 2 jours en PBS.

Un marquage à l'iodure de propidium (IP), un marqueur de l'ADN, est réalisé par addition sur le culot cellulaire (dans l'ordre) de 100 µl de RNase (soit 50 UI) et 250 µl d'IP à 50 µg/ml.

On effectue la lecture au cytomètre dans les 15 min.

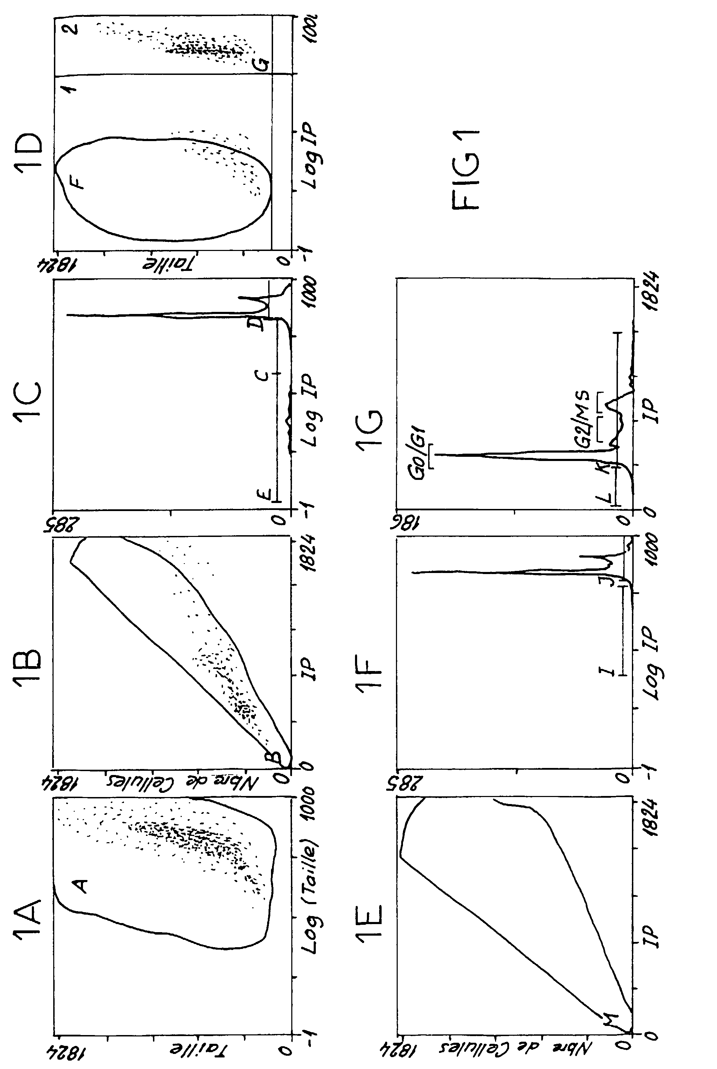

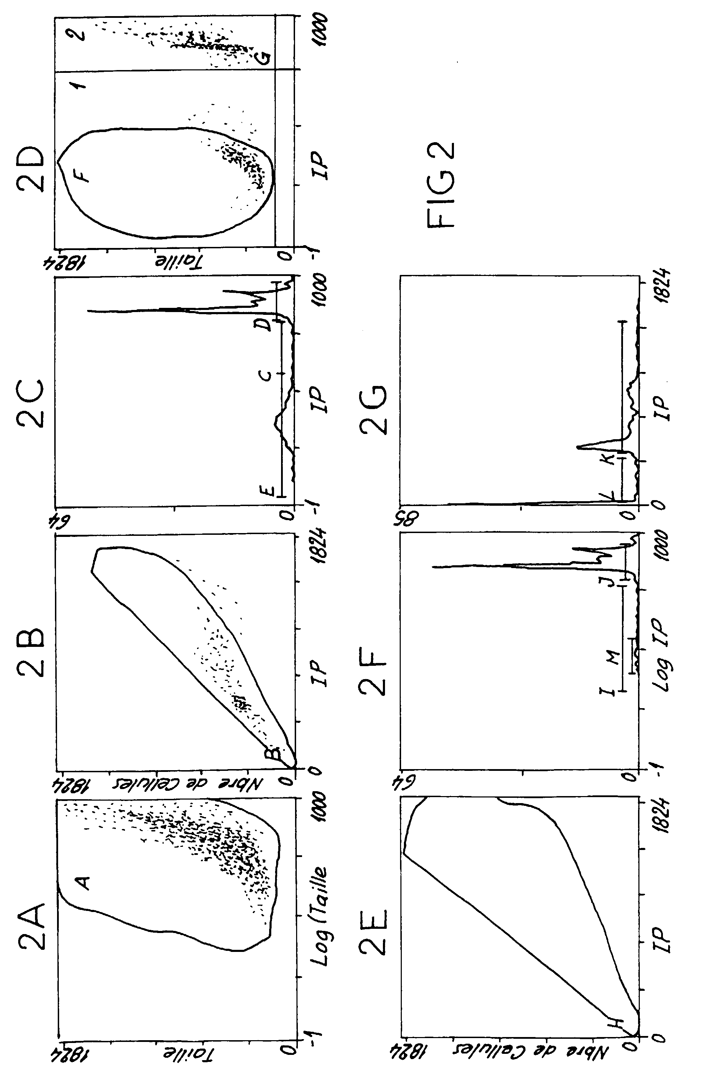

Le marquage à l'IP, éventuellement associé à un marquage GFAP, est utilisé pour identifier et localiser les astrocytes. L'analyse de chaque échantillon est réalisée sur 3000 cellules (cf figures 1A, 1B, 1C et 2A, 2B, 2C).

Par cette méthode les noyaux marqués à l'IP des cellules vivantes sont visualisés sous forme de 3 pics de fluorescence représentant respectivement les phases G0/G1, S et G2/M du cycle cellulaire, comme montré sur la figure 1G. Au contraire, les noyaux marqués à l'IP des cellules apoptotiques, dont l'ADN fragmenté a été extrait au préalable, sont visualisés sous forme d'un pic unique relativement diffus positionné en sub-G1, comme montré sur la figure 2G. Pour démontrer que le pic sub-diploïde (sub-G1) est uniquement dû aux cellules apoptotiques, et pour éliminer des éléments interférents (cellules nécrotiques et débris), les cellules sont tout d'abord analysées dans des conditions expérimentales permettant d'induire une mort par nécrose (Wyllie et al., J. Pathol., 1984, 142, 67-77) (cf figures 1D, IE et 2D, 2E). Ceci permet d'ajuster les réglages du cytomètre de façon à s'affranchir des cellules non apoptotiques. Les cellules apoptotiques sont alors analysées comme décrit précédemment (cf figures 1F et 2F sur lesquelles on observe les cellules mortes par apoptose).

Les résultats obtenus sont rassemblés dans le tableau I présenté en fin de description.

Exemple 2 : Etape de détermination de cytotoxicité sur la lignée d'astrocytes immortalisés, constituant notamment l'étape de détection de la viabilité cellulaire

Comme indiqué dans la description, on peut avantageusement pratiquer une étape de détection de la viabilité cellulaire, avant l'étape de détection par cytométrie de flux. Juqu'au cinquième jour, le protocole expérimental décrit dans l'exemple 1 est répété.

Cinquième jour :

La quantité de cellules vivantes restant dans les puits est évaluée par incorporation de MTT (bromure de tétrazolium) puis lyse à l'isopropanol acide pour mettre en évidence les cristaux de formazan obtenus après incorporation de MTT dans les cellules vivantes.

On prépare une solution de MTT à 0,5 mg/ml en DMEM/F12 par dilution du MTT (congelé) au 1/10ème dans le DMEM/F12.

Les boîtes sont sorties de l'étuve. Le film protecteur est ôté. Le milieu est aspiré en prenant soin de ne pas aspirer les cellules adhérentes au fond des puits. On additionne 250 µl/puits de la solution de MTT (en DMEM/F12). On laisse incuber 3 heures au moins à l'étuve (37°C).

On prépare l'isopropanol acide (addition de 40 µl/ml d'HCl 1N dans l'isopropanol pur) en prévoyant 100 µl/ puits pour les plaques 48 puits.

A titre d'exemple, pour 4 plaques 48 puits, il faut 48 x 4 x 100, soit environ 20 ml d'isopropanol acide.

On prépare 30 ml d'isopropanol + 1,2 ml HCl 1N.

On sort les plaques de l'étuve, on aspire la solution de MTT puis on ajoute 100 µl/puits d'isopropanol acide.

Si la coloration est trop foncée, on rajoute 50 µl/puits d'isopropanol acide.

La solution ainsi obtenue dans chaque puits est transférée dans une plaque 96 puits pour pouvoir être lue sur le lecteur de microplaques. On reprend 70 µl/puits d'une plaque 48 puits pour transférer dans une plaque 96 puits.

Les DO sont lues à 570 nm (ref 630 nm).

Les valeurs obtenues sont interprétées sur un logiciel approprié, afin d'obtenir un % de gliotoxicité :

Pour chaque boîte, sur l'ensemble des puits contenant les cellules ayant poussé normalement (100% viabilité) on calcule la moyenne et l'écart type des DO lues, le "cut off" est obtenu par différence :

$$\text{cut off} = \text{moyenne} - 2\text{x écart-type} \ (= CO).$$

Chaque échantillon testé est représenté par 3 DO (correspondant aux 3 puits dans lesquels 10 ou 20 µl d'échantillon ont été ajoutés), on calcule la moyenne des 3 DO (= DOc) ; ainsi pour chaque échantillon on obtient une différence de DO (DO) et un pourcentage de toxicité (% tox) :

$$DO = CO - DOc$$

$$\% \text{ tox} = (1 - (DOc / CO)) \text{ x } 100$$

Si pour un échantillon les 3 puits donnent 3 DO différentes, l'échantillon est retesté.

Exemple 3 : Techniques d'enrichissement de l'échantillon biologique, en facteur gliotoxique, pour obtenir une fraction, soumise à l'étape selon l'Exemple 1, et, éventuellement l'étape de détermination selon l'Exemple 2

3.1) Précipitation de la fraction protéique

On utilise une solution à 60 % en poids de sulfate d'ammonium dans l'eau.

3.2) Chromatographie par exclusion

On utilise une colonne TSK G 2000 (commercialisée par SUPELCO), en phase liquide haute pression, avec un tampon de pH à 6,8, avec un phosphate à 0,1 M, et du sulfate de sodium à 0,1 M.

Le temps d'acquisition est de 60 minutes, et l'échantillon injecté est de 1 ml.

3.3) Chromatographie par échange ionique

On utilise une colonne DEAE-Sepharose (DEAE: diéthylaminoéthyl) (commercialisée par PHARMACIA), en phase liquide haute pression, à débit rapide, avec un tampon Tris 20 mM, à un pH de 7,5, avec une élution par étapes avec une solution de NaCl de 100 mM à 1 M, la fraction utile étant à 200 mM.

3.4) Electrophorèse à une ou deux dimensions

On utilise la technique SDS PAGE(PolyAcrylamide Gel Electrophoresis), avec un gel à 17 % d'acrylamide. On transfère l'électrogramme sur membrane Porablot (nom commercial), et on procède à une coloration avec un colorant noir-amide.

Exemple 4 : Equivalence biologique de l'urine et du LCR d'un patient atteint de la sclérose en plaques, pour la détermination du facteur gliotoxique.

4.1) Des échantillons de LCR d'une part, et d'urine d'autre part, de patients souffrant de la sclérose en plaques (SEP), sont soumis à volume égal à un même prétraitement de séparation de la fraction protéique, selon l'exemple 3.1, laquelle est ensuite soumise et à une chromatographie par exclusion selon l'exemple 3.2, et à une chromatographie par échange ionique selon l'exemple 3.3.

Pour chaque type de chromatographie, l'urine et le LCR sont résolus de la même manière, et laissent apparaître une fraction, située entre 15 kD et 20 kD en ce qui concerne la chromatographie par exclusion, et éluée par 200 mM de NaCl en ce qui concerne la chromatographie par échange d'ion.

Cette fraction est testée selon l'étape décrit à l'exemple 2, et démontre une activité gliotoxique.

4.2) Des échantillons de LCR d'une part, et d'urine d'autre part, de patients SEP, sont soumis à une électrophorèse à une dimension, selon l'exemple 3.4. Dans les deux cas, on observe un seuil de coupure situé entre 15 et 25 kD, correspondant à une activité cytotoxique.

4.3) Des échantillons d'urine de patients SEP, et de patients sains, et de patients atteints d'autres maladies neurologiques, sont soumis à l'étape selon l'exemple 2.

Les résultats rassemblés selon le tableau II (figurant en fin de description) montrent une relative spécificité de l'urine en tant qu'échantillon biologique pour la détermination du facteur gliotoxique.

Exemple 5 : Détermination par voie urinaire du facteur gliotoxique dans une population importante, par détection de cytotoxicité .

Les échantillons urinaires sont testés selon le protocole identifié dans l'exemple 2.

En ce qui concerne les patients atteints de sclérose en plaques, il s'agit de patients hospitalisés dans un service de neurologie (-90 % pour un flash corticoïde au moment d'une poussée), ou dans un service de rééducation spécialisé (patients généralement hors poussées, -50 % en phase chronique de la maladie). En ce qui concerne les patients atteints d'autres maladies, il s'agit de patients hospitalisés dans un service de neurologie (sans restriction de pathologie, d'âge ou de sexe). Les résultats obtenus sur 108 urines de patients atteints de sclérose en plaques (classés selon les critères de Poser certaines ou probables, C.M. Poser et al., New diagnostic criteria for multiple sclerosis : guidelines for research protocols, dans "The Diagnosis of Multiple Sclerosis", C.M. Poser, D.W. Paty, L. Scheinberg, W.I. Mac Donald, G.C. Ebers, pp 225-229, 1984, Thieme Stratton Inc., New-York), 116 urines de patients atteints d'autres maladies neurologiques et 29 urines de témoins sains sont présentés dans le Tableau III (figurant en fin de description).

Les résultats obtenus mettent en évidence une positivité du test chez 98 patients atteints de sclérose en plaques et un test négatif chez 10 patients atteints de sclérose en plaques. Mais cependant, ce test génère 40 faux-positifs dans les patients atteints d'autres maladies neurologiques et 2 faux-positifs dans les contrôles sains.

Le test défini dans l'exemple 2 n'est donc pas totalement spécifique à lui seul.

Exemple 6 : Détermination de l'état des cellules par cytométrie

La méthode selon l'Exemple 1 permet à la fois de juger de l'état des astrocytes et de détecter la mort cellulaire (notamment différenciation entre nécrose et apoptose), et la ploïdie.

Cette méthode est basée sur la détection par cytométrie de flux (CMF) i) de la morphologie cellulaire (le FALS ("Forward Light Angle Scatter") permet la visualisation de la taille des cellules, le SS ("Side Scatter") permet la visualisation de leur structure), ii) des caractéristiques astrocytaires (marquage anti-GFAP ("Glial Fibrillary Acidic Protein"), révélation anti-Ig-FITC), iii) de la ploïdie (marquage à l'iodure de propidium (IP) après fixation à l'éthanol 70 % et extraction des éventuels fragments d'ADN) et par suite du cycle cellulaire. Cette dernière étape (utilisant l'IP) permet de différencier les débris cellulaires, les cellules nécrotiques, les cellules apoptotiques et les cellules diploïdes capables de se diviser normalement.

On a alors testé 30 faux-positifs obtenus selon l'Exemple 5, et fait la constatation que les urines non-SEP produisaient des effets de nécrose et/ou inhibition de prolifération, à l'exclusion de phénomènes apoptotiques, alors que les urines SEP conduisaient au moins à une apoptose.

Exemple 7 : Association des méthodes selon les exemples 1 et 2

L'association des deux tests a alors été évaluée sur 30 urines de sclérose en plaques et 30 urines de témoins neurologiques (non-SEP) après l'étape de détection de la viabilité cellulaire. L'étude a conduit à l'obtention du tableau IV, figurant à la fin de la description. Ainsi les résultats obtenus montrent une bonne sensibilité et une bonne spécificité.

Tableau I : Résultats obtenus par le procédé de l'invention tel que décrit dans l'exemple 1, par cytométrie de flux après extraction des fragments d'ADN et coloration de l'ADN à l'iodure de propidium

|  | SEP certaines | Autres maladies neurologiques | Témoins sains |
|---|---|---|---|
| nb de patients testés | 35 | 36 | 15 |
| Apoptose | 32 soit 91% | 5 soit 14% | 0 soit 0% |
| Absence d'apoptose | 3 soit 9% | 31 soit 89% | 15 soit 100% |

Tableau II :

| Test de gliotoxicité sur culture astrocytaire à partir d'urines (cf exemple 4) | | | |
|---|---|---|---|
| Test MTT | SEP (n=30) | AMN* (n=32) | contrôles sains (n=19) |
| positif | n=28 | n=5 | n=1 |
| négatif | n=2 | n=27 | n=18 |
| AMN : autres maladies neurologiques, non-SEP | | | |

Tableau III:

| Test de gliotoxicité sur culture astrocytaire à partir d'urines (cf exemple 5) | | | |
|---|---|---|---|
| Test MTT | SEP (n=108) | AMN* (n=116) | contrôles sains (n=29) |
| positif | n=98 91% | n=40 34% | n=2 7% |
| négatif | n=10 9% | n=76 66% | n=27 93% |
| AMN : autres maladies neurologiques, non-SEP | | | |

Tableau IV:

| Test de gliotoxicité sur culture astrocytaire à partir d'urines (cf Exemple 7) | | |
|---|---|---|
| Test par CMF** | SEP (n=30) | AMN* (n=30) |
| nécrose ou inhibition de prolifération | n=1 3% | n=30 100% |
| apoptose | n=29 97% | n=0 0% |
| AMN : autres maladies neurologiques, non-SEP CMF : cytométrie de flux | | |

**EP 0 925 504 B1**

**Revendications**

1.  Procédé pour détecter et/ou quantifier, dans un échantillon biologique, l'activité gliotoxique d'un facteur gliotoxique vis-à-vis de cellules macrogliales cibles, **caractérisé en ce que** l'échantillon biologique est un échantillon d'urine.

2.  Procédé pour détecter et/ou quantifier, dans un échantillon biologique, un facteur cytotoxique vis-à-vis de cellules macrogliales adhérentes cibles et dont la cytotoxicité induit la mort par apoptose desdites cellules, selon lequel on dispose d'une fraction de départ dudit échantillon, éventuellement enrichie en ledit facteur toxique par un traitement préalable, on incube ladite fraction de départ avec un milieu de culture de référence comprenant des cellules adhérentes cibles, et on détecte et/ou quantifie les cellules adhérentes mortes par apoptose, **caractérisé en ce que,** aux fins de la détection et/ou quantification des cellules adhérentes mortes par apoptose, on détecte, par cytométrie de flux, au moins une caractéristique directe ou indirecte, associée aux cellules adhérentes apoptotiques de tout ou partie du milieu incubé, qui, si elle existe et/ou est quantifiée, qualifie ledit échantillon biologique comme positif, c'est-à-dire contenant ledit facteur toxique, ledit échantillon étant un échantillon d'urine.

3.  Procédé selon la revendication 2, caractérisé en ce que les cellules macrogliales adhérentes cibles sont des cellules astrocytaires.

4.  Procédé selon la revendication 2 ou 3, caractérisé en ce que, aux fins de la détection et/ou quantification des cellules macrogliales mortes par apoptose spécifiquement, par cytométrie de flux, le procédé comprend les étapes suivantes :

    -   le décollement des cellules macrogliales adhérentes,
    -   le traitement des cellules ainsi décollées par un agent de fixation cellulaire et de perméabilisation de la membrane cytoplasmique desdites cellules,
    -   l'extraction de fragments d'ADN intracellulaire résultant de l'apoptose,
    -   le marquage de l'ADN cellulaire restant, par un marqueur approprié, et
    -   la détection par cytométrie de flux de la ploïdie des cellules macrogliales.

5.  Procédé selon la revendication 4, caractérisé en ce que l'agent de fixation cellulaire et de perméabilisation de la membrane cytoplasmique des cellules est l'éthanol.

6.  Procédé selon la revendication 4 ou 5, caractérisé en ce que le marqueur de l'ADN est l'iodure de propidium (IP).

7.  Procédé selon la revendication 2 ou 3, caractérisé en ce que, aux fins de la détection et/ou quantification des cellules macrogliales mortes par apoptose spécifiquement, par cytométrie de flux, le procédé comprend les étapes suivantes :

    -   l'induction d'une nécrose sur un échantillon de cellules macrogliales vivantes, différent du milieu d'incubation contenant la fraction de départ et le milieu de culture de référence,
    -   le marquage des débris d'ADN résultant de la nécrose, lesdits débris étant associés à des fragments de la membrane cytoplasmique des cellules macrogliales nécrotiques, le marquage étant effectué après décollement des cellules,
    -   la localisation en cytométrie de flux, des cellules mortes par nécrose, et réglage approprié du cytomètre permettant lors de l'étape de détection des cellules mortes par apoptose, d'exclure les cellules mortes par nécrose,
    -   la détection dans l'échantillon à analyser des cellules mortes par apoptose.

8.  Procédé selon la revendication 2 ou 3, caractérisé en ce que, aux fins de la détection et/ou quantification des cellules macrogliales mortes par apoptose spécifiquement, par cytométrie de flux, ledit procédé comprend les étapes suivantes :

    -   le partage des cellules du milieu d'incubation contenant la fraction de départ et le milieu de culture de référence, en deux parties égales, et le décollement des cellules,
    -   la détection, par cytométrie de flux, dans une des deux parties, des cellules macrogliales mortes par apoptose ou par nécrose, d'une part, et/ou des cellules macrogliales vivantes, d'autre part, étape selon laquelle on traite ladite fraction avec un agent de fixation cellulaire et de perméabilisation de la membrane cytoplasmique ; on extrait les fragments d'ADN ; on marque à l'aide d'un marqueur de l'ADN intracellulaire restant, et on détecte ce dernier,

**11**

- la détection, par cytométrie de flux après fixation sans extraction, dans l'autre des deux parties, des cellules macrogliales vivantes et des cellules macrogliales apoptotiques, d'une part, et/ou des cellules macrogliales nécrotiques, d'autre part,
- la déduction des détections effectuées sur chacune des deux parties, de la quantité de cellules macrogliales apoptotiques.

9. Procédé selon la revendication 2 ou 3, caractérisé en ce qùe le milieu de culture de référence comprend une lignée cellulaire immortalisée de cellules macrogliales, par exemple une lignée cellulaire immortalisée d'astrocytes.

10. Procédé selon l'une quelconque des revendications 1 à 9, caractérisé en ce qu'il comprend, avant l'étape de détection et/ou quantification des cellules macrogliales apoptotiques, une étape de détection de la viabilité cellulaire, selon laquelle on inocule la fraction de départ dans le milieu de culture de référence, puis on observe le taux de prolifération des cellules macrogliales par rapport à une valeur de référence, en deçà de laquelle ladite fraction dite positive est retenue pour être soumise ensuite à l'étape de détection de l'apoptose des cellules macrogliales spécifiquement, pour qualifier ou non l'échantillon biologique comme vrai positif.

11. Procédé selon la revendication 10, caractérisé en ce que pendant l'étape de détection de la viabilité cellulaire le taux de prolifération des cellules macrogliales est obtenu par coloration mitochondriale des cellules macrogliales du milieu de culture, par exemple par du bromure de méthyltétrazolium, puis par mesure de la densité optique du milieu de culture coloré.

12. Procédé selon l'une quelconque des revendications 2 à 11, caractérisé en ce qu'on pratique en outre, pendant l'étape de détection de l'apoptose des cellules macrogliales spécifiquement, une détermination de la présence et/ ou quantité d'une protéine permettant de discriminer les sous-populations cellulaires, telle que la protéine acide fibrillaire gliale (GFAP), par exemple avec un anticorps anti-GFAP identifiable directement ou indirectement.

13. Procédé selon l'une quelconque des revendications précédentes, caractérisé en ce que la fraction de départ est obtenue par enrichissement ou purification de l'échantillon biologique en facteur gliotoxique, selon au moins l'un des traitements suivants, à savoir précipitation de la fraction protéique, par 5 exemple avec du sulfate d'ammonium, chromatographie par exclusion et/ou ionique, électrophorèse à une ou deux dimensions, mise en contact avec la protéine A ou une lectine, par exemple la concanavaline-A.

14. Utilisation d'un échantillon d'urine pour y LO détecter un facteur gliotoxique tel qu'associé à la sclérose en plaques.

**Claims**

1. Process for detecting and/or quantifying, in a biological sample, the gliotoxic activity of a factor which is gliotoxic for target macroglial cells, characterized in that the biological sample is a urine sample.

2. Process for detecting and/or quantifying, in a biological sample, a factor which is cytotoxic for target adherent macroglial cells and whose cytotoxicity induces the death of the said cells by apoptosis, according to which process a starting fraction of the said sample, where appropriate enriched in the said toxic factor by a prior treatment, is to hand, the said starting fraction is incubated with a reference culture medium comprising target adherent cells and the adherent cells which have died by apoptosis are detected and/or quantified, characterized in that, for the purpose of detecting and/or quantifying the adherent cells which have died by apoptosis, at least one direct or indirect feature which is associated with the apoptotic adherent cells of all or part of the incubated medium and which, if it exists and/or is quantified, categorizes the said biological sample as being positive, that is to say containing the said toxic factor, is detected by flow cytometry, with the said sample being a urine sample.

3. Process according to claim 2, characterized in that the target adherent macroglial cells are astrocytic cells.

4. Process according to claim 2 or 3, characterized in that, for the purpose of specifically detecting and/or quantifying, by flow cytometry, the macroglial cells which have died by apoptosis, the process comprises the following steps:

- detaching the adherent macroglial cells,
- treating the cells which have thus been detached with an agent for cell fixation and permeabilization of the cytoplasmic membrane of the said cells,

- extracting intracellular DNA fragments resulting from the apoptosis,
- labelling the remaining cellular DNA with an appropriate label, and
- detecting, by flow cytometry, the ploidy of the macroglial cells.

**5.** Process according to claim 4, characterized in that the agent for cell fixation and permeabilization of the cytoplasmic membrane of the cells is ethanol.

**6.** Process according to claim 4 or 5, characterized in that the label for the DNA is propidium iodide (PI).

**7.** Process according to claim 2 or 3, characterized in that, for the purpose of specifically detecting and/or quantifying, by flow cytometry, the macroglial cells which have died by apoptosis, the process comprises the following steps:

- inducing a necrosis in a sample of living macroglial cells which is different from the incubation medium containing the starting fraction and the reference culture medium,
- labelling the DNA debris which result from the necrosis, with the said debris being associated with fragments of the cytoplasmic membrane of the necrotic macroglial cells, and with the labelling being effected after detaching the cells,
- locating, in flow cytometry, the cells which have died by necrosis and appropriately adjusting the cytometer, thereby enabling the cells which have died by necrosis to be excluded during the step of detecting the cells which have died by apoptosis,
- detecting the cells which have died by apoptosis in the sample to be analysed.

**8.** Process according to claim 2 or 3, characterized in that, for the purpose of specifically detecting and/or quantifying, by flow cytometry, the macroglial cells which have died by apoptosis, the said process comprises the following steps:

- dividing the cells of the incubation medium containing the starting fraction and the reference culture medium into two equal parts and detaching the cells,
- detecting, by flow cytometry, in one of the two parts, the macroglial cells which have died by apoptosis or by necrosis, on the one hand, and/or the living macroglial cells, on the other hand, in which step the said fraction is treated with an agent for cell fixation and for permeabilization of the cytoplasmic membrane; the DNA fragments are extracted; the remaining intracellular DNA is labelled with a marker and this DNA is detected,
- detecting, by flow cytometry after fixation without extraction, in the other of the two parts, the living macroglial cells and the apoptotic macroglial cells, on the one hand, and/or the necrotic macroglial cells, on the other hand,
- deducing, from the detections effected on each of the two parts, the quantity of apoptotic macroglial cells.

**9.** Process according to claim 2 or 3, characterized in that the reference culture medium comprises an immortalized cell line of macroglial cells, for example an immortalized astrocyte cell line.

**10.** Process according to any one of claims 1 to 9, characterized in that it comprises, before the step of detecting and/ or quantifying the apoptotic macroglial cells, a step of detecting cell viability, according to which the starting fraction is inoculated into the reference culture medium and the rate of proliferation of the macroglial cells is compared with a reference value, below which the said fraction, which is said to be positive, is retained for then being subjected to the step of specifically detecting the apoptosis of the macroglial cells, in order to categorize or not categorize the biological sample as being truly positive.

**11.** Process according to claim 10, characterized in that, during the step of detecting cell viability, the rate of proliferation of the macroglial cells is obtained by staining the mitochondria of the macroglial cells of the culture medium, for example with methyltetrazolium bromide, and then measuring the optical density of the stained culture medium.

**12.** Process according to any one of claims 2 to 11, characterized in that, during the step of specifically detecting the apoptosis of the macroglial cells, the presence and/or quantity of a protein, such as the glial fibrillary acidic protein (GFAP), which makes it possible to discriminate between subpopulations of cells is additionally determined, for example using an anti-GFAP antibody which can be identified directly or indirectly.

**13.** Process according to any one of the preceding claims, characterized in that the starting fraction is obtained by enriching or purifying the biological sample in gliotoxic factor using at least one of the following treatments, namely precipitation of the protein fraction, for example with ammonium sulphate, exclusion and/or ion exchange chromatography, one-dimensional or two-dimensional electrophoresis, or bringing into contact with protein A or a lectin, for example concanavalin A.

14. Use of a urine sample for detecting in it a gliotoxic factor such as associated with multiple sclerosis.

**Patentansprüche**

1. Verfahren, um in einer biologischen Probe die gegenüber Makrogliazielzellen qliotoxische Aktivität eines gliotoxischen Faktors nachzuweisen und/oder zu quantifizieren, dadurch gekennzeichnet, daß die biologische Probe eine Urinprobe ist.

2. Verfahren, um in einer biologischen Probe einen gegenüber adhärenten Makrogliazielzellen cytotoxischen Faktor nachzuweisen und/oder zu quantifizieren, dessen Cytoxizität das Absterben dieser Zellen durch Apoptose induziert, bei dem eine Ausgangsfraktion dieser Probe bereitgestellt wird, die eventuell durch vorherige Behandlung bezüglich des toxischen Faktors angereichert ist, die Ausgangsfraktion mit einem Referenzkulturmedium inkubiert wird, das adhärente Zielzellen aufweist, und die durch Apoptose abgestorbenen adhärenten Zellen detektiert und/oder quantifiziert werden, dadurch gekennzeichnet, daß zum Nachweis und/oder zur Quantifizierung der durch Apoptose abgestorbenen adhärenten Zellen mittels Fluxcytometrie zumindest ein direktes oder indirektes Merkmal nachgewiesen wird, das mit apoptotischen adhärenten Zellen des gesamten oder eines Teils des inkubierten Mediums assoziiert ist, und das, wenn es existiert und/oder quantifiziert wird, die biologische Probe als positiv d.h. als den toxischen Faktor enthaltend kennzeichnet, wobei die Probe eine Urinprobe ist.

3. Verfahren nach Anspruch 2, dadurch gekennzeichnet, daß die adhärenten Makrogliazielzellen astrocytäre Zellen sind.

4. Verfahren nach Anspruch 2 oder 3, dadurch gekennzeichnet, daß zum Nachweis und/oder zur Quantifizierung der durch spezifische Apoptose abgestorbenen Makrogliazellen mittels Fluxcytometrie das Verfahren die folgenden Schritte aufweist:

   - Ablösung der adhärenten Makrogliazellen;

   - Behandlung der so abgelösten Zellen mit einer Substanz zur zellulären Fixierung und Permeabilisierung der Cytoplasmamembran der Zellen;

   - Extraktion von aus der Apoptose resultierenden intrazellulären DNA-Fragmenten;

   - Markierung der noch vorhandenen zellulären DNA durch einen geeigneten Marker, und

   - Nachweis der Ploidie der Makrogliazellen mittels Fluxcytometrie.

5. Verfahren nach Anspruch 4, dadurch gekennzeichnet, daß die Substanz zur zellulären Fixierung und Permeabilisierung der Cytoplasmamembran der Zellen Ethanol ist.

6. Verfahren nach Anspruch 4 oder 5, dadurch gekennzeichnet, daß der Marker der DNA Propidiumjodid ist (PJ).

7. Verfahren nach Anspruch 2 oder 3, dadurch gekennzeichnet, daß zum Nachweis und/oder zur Quantifizierung der durch spezifische Apoptose abgestorbenen Makrogliazellen mittels Fluxcytometrie das Verfahren die folgenden Schritte aufweist:

   - Induktion einer Nekrose auf einer Probe lebender Makrogliazellen, die sich von dem Inkubationsmedium, das die Ausgangsfraktion und das Referenzkulturmedium enthält, unterscheidet;

   - Markierung der aus der Nekrose resultierenden DNA-Trümmer, wobei die Trümmer mit Fragmenten der Cytoplasmamembran der nekrotischen Makrogliazellen assoziiert sind, und die Markierung nach Ablösung der Zellen durchgeführt wird;

   - Lokalisation der durch Nekrose abgestorbenen Zellen mittels Fluxcytometrie und geeignete Einstellung des Cytometers, so daß bei dem Schritt des Nachweisens der durch Apoptose abgestorbenen Zellen der Ausschluß der durch Nekrose abgestorbenen Zellen möglich ist;

- Nachweis der durch Apoptose abgestorbenen Zellen in der zu analysierenden Probe.

**8.** Verfahren nach Anspruch 2 oder 3, dadurch gekennzeichnet, daß zum Nachweis und/oder zur Quantifizierung der durch spezifische Apoptose abgestorbenen Makrogliazellen mittels Fluxcytometrie das Verfahren die folgenden Schritte aufweist:

- Aufteilung der Zellen des Inkubationsmediums, das die Ausgangsfraktion und das Referenzkulturmedium enthält, in zwei gleiche Teile, und Ablösung der Zellen;

- Nachweis in einem der beiden Teile mittels Fluxcytometrie einerseits von durch Apoptose oder Nekrose abgestorbenen Makrogliazellen und/oder andererseits von lebenden Makrogliazellen, ein Schritt, bei dem die Fraktion mit einer Substanz zur zellulären Fixierung und Permeabilisierung der Cytoplasmamembran behandelt wird; Extrahieren der DNA-Fragmente; Markieren intrazellulär verbleibender DNA mittels eines Markers, und Nachweisen des letzteren;

- Nachweis in dem anderen der beiden Teile mittels Fluxcytometrie nach Fixierung ohne Extraktion einerseits von lebenden Makrogliazellen und apoptotischen Makrogliazellen und/oder andererseits von nekrotischen Makrogliazellen, und

- Schlußfolgerung aus den für jeden der beiden Teile erfolgten Nachweisen auf die Menge der apoptotischen Makrogliazellen.

**9.** Verfahren nach Anspruch 2 oder 3, dadurch gekennzeichnet, daß das Referenzkulturmedium eine immortalisierte Zellinie von Makrogliazellen aufweist, z.B. eine immortalisierte Astrocytenzellinie.

**10.** Verfahren nach einem der Ansprüche 1 bis 9, dadurch gekennzeichnet, daß es vor dem Schritt des Nachweisens und/oder Quantifizierens der apoptotischen Mikrogliazellen einen Schritt umfaßt, um die zelluläre Lebensfähigkeit nachzuweisen, bei dem die Ausgangsfraktion in das Referenzkulturmedium eingeimpft wird, dann der Proliferationsgrad der Makrogliazellen im Vergleich zu einem Referenzwert beobachtet wird, diesseits dessen eine als positiv bezeichnete Fraktion zurückbehalten wird, um sie anschließend einem Schritt zum Nachweis makrogliazellspezifischer Apoptose zu unterziehen, um zu qualifizieren, ob die biologische Probe wirklich positiv ist oder nicht.

**11.** Verfahren nach Anspruch 10, dadurch gekennzeichnet, daß während des Schrittes des Nachweisens der zellulären Lebensfähigkeit der Proliferationsgrad der Makrogliazellen durch mitochondriale Anfärbung der Makrogliazellen des Kulturmediums, z.B. durch Methyltetrazoliumbromid, und danach durch Messung der optischen Dichte des gefärbten Kulturmediums erhalten wird.

**12.** Verfahren nach einem der Ansprüche 2 bis 11, dadurch gekennzeichnet, daß zusätzlich während des Schrittes des Nachweisens der makrogliazellspezifischen Apoptose eine Bestimmung des Vorliegens und/oder eine Quantifizierung eines Proteins durchgeführt wird, das es erlaubt, die zellulären Unterpopulationen zu unterscheiden, wie z.B. das saure Gliafaserprotein (GFAP), z.B. mit einem direkt oder indirekt identifizierbaren Anti-GFAP-Antikörper.

**13.** Verfahren nach einem der vorherigen Ansprüche, dadurch gekennzeichnet, daß die Ausgangsfraktion durch Anreicherung oder Aufreinigung des gliotoxischen Faktors in bzw. aus der biologischen Probe nach zumindest einer der nachfolgenden Behandlungen erhalten wird: Präzipitation der Proteinfraktion, z.B. mit Ammoniumsulfat, Ausschluß- und/oder Ionenchromatographie, ein- oder zweidimensionale Elektrophorese, in-Kontakt-bringen mit Protein A oder einem Lektin, z.B. dem Concanavalin-A.

**14.** Verwendung einer Urinprobe, um dort einen solchen gliotoxischen Faktor nachzuweisen, der mit multipler Sklerose assoziiert ist.

FIG 1

EP 0 925 504 B1

FIG 2

2A

2B

2C

2D

2E

2F

2G

EP 0 925 504 B1